# EUROPEAN PATENT SPECIFICATION

(11) **EP 1 224 536 B1**
(45) Date of publication and mention of the grant of the patent: **21.12.2005**
(21) Application number: 00936432.4
(22) Date of filing: 30.05.2000
(51) Int. Cl.: G06F 9/00

(54) **CONTEXT MANAGEMENT SERVER APPLIANCE**
SERVER ZUM VERWALTEN VON KONTEXTINFORMATION
DISPOSITIF SERVEUR DE GESTION CONTEXTUELLE

(30) Priority: 28.05.1999 US 136670 P; 14.06.1999 US 139235 P
(43) Date of publication of application: 24.07.2002
(73) Proprietor: Sentillion, Inc., Andover, MA 01810 (US)
(72) Inventor: SELIGER, Robert, Winchester, MA 01890 (US)
(74) Representative: HOFFMANN EITLE
(86) International application number: PCT/US2000/014942
(87) International publication number: WO 2001/011464

(56) References cited:
- EP-A- 0 459 912
- EP-A- 0 540 151
- EP-A- 0 803 808
- WO-A-91/12583
- WO-A-95/03580
- GB-A- 2 305 087
- GB-A- 2 305 747
- GB-A- 2 323 458
- CLINICAL CONTEXT WORKING GROUP: "The Clinical Context Object Workgroup: Its Standard and Methods" INTERNET DOCUMENT: CLINICAL CONTEXT OBJECT WORKING GROUP WHITE PAPER, [Online] 16 February 1998 (1998-02-16), XP002154044 Retrieved from the Internet: <URL:http://www.hl7.org/library/committees /sigvi/CCOW_white_paper.doc> [retrieved on 2000-11-23]
- RIC JACOBONI: "Les fichiers d'initialisation de Linux" INTERNET DOCUMENT, [Online] 20 October 1998 (1998-10-20), XP002154045 Retrieved from the Internet: <URL:http://www.linux-france.org/article/s ys/init-jaco/> [retrieved on 2000-11-22]
- V. JAGANNATHAN, KENT WREDER, ROBERT GLICKSMAN, YASSER ALSAFADI: "Objects in Healthcare- Focus on Standards" STANDARD VIEW, ACM, 1 March 1998 (1998-03-01), pages 22-26, XP002154046
- TRISHA COLEMAN, KATHY NEAL : "DUKE UNIVERSITY HEALTH SYSTEM HOSTS NEW RESEARCH CENTER SEEKING VISUAL INTEGRATION OF HEALTHCARE APPLICATIONS" INTERNET DOCUMENT: ABOUT VIRC: PRESS RELEASES, [Online] 19 February 1999 (1999-02-19), XP002154047 Retrieved from the Internet: <URL:http://www.visualintegration.org/abou t/press/021999.html> [retrieved on 2000-11-22]

## Description

### BACKGROUND OF THE INVENTION

### CROSS-REFERENCE TO RELATED APPLICATIONS

The present application claims domestic priority under 35 U.S.C. §119(e) to U.S. Patent Application Serial No. 60/136,670, filed May 28, 1999, and to U.S. Patent Application Serial No. 60/139,235, filed June 14, 1999, both of which are incorporated herein by reference. The subject matter disclosed herein is also related to the subject matter of U.S. Patent Application Serial No. 09/545,396, filed April 7, 2000, also incorporated herein by reference.

### FIELD OF THE INVENTION

The present invention relates to context management systems. More particularly, the invention relates to systems in which context management software is configured as a server. Yet more particularly, the invention relates to a server appliance including a standalone computer and software system, which performs context management over a network. In some aspects, the invention relates to such a server appliance, which is applicable to context management of healthcare applications.

### RELATED ART

There are many businesses or fields of endeavor, which rely on the use of plural desktop computer applications. One such field is the modem practice of medicine. In such a setting, users quite often find themselves entering and reentering similar information over and over. For example, a single user may have to repeat login information in plural applications, followed by the same or similar client information. Such information that defines the environment in which each application operates is known as context. That is, context is a collection of data items and corresponding values, wherein the items represent information required in common between plural applications in an industry or business setting. For example, in health care, a patient identifier (patient ID) is an item which is part of the context in which plural clinical applications may participate, or share.

In the modem practice of medicine, a physician or other professional or staff member may need to store, retrieve, analyze, etc. various types of patient data. The patient data to be processed may be clinical; e.g. x-ray images or blood work results, or may be financial, e.g. insurance cover and billing history. Thus, clinical applications, such as those to store, retrieve and display x-ray images and those to store, retrieve and display blood work results have inputs and outputs which fall into two broad classes: highly specialized, work product specific I/O; and more general, context-related I/O.

The desirability of managing context information, so that a user at a workstation need not reenter information such as user identification (user ID) or patient identification (patient ID) has long been recognized.

A standard known as Health Level Seven Context Management Specification Version CM-1.1 was promulgated by the Health Level Seven (HL7) Clinical Context Object Workgroup (CCOW) on November 6, 1999, incorporated herein in its entirety by reference, to define an interface and other architectural definitions of a Context Management Architecture (CMA), whereby clinical applications interact with a Context Manager to manage context information across a range of clinical and other health care related applications.

At this time, there is no other known, comprehensive context management software packages available. Some small steps have been taken for example to share context amongst one publisher's own titles, using proprietary methods absent a context manager, or to permit a user to sign onto a single application which transfers user context to plural other applications. However, no context manager handling both user and patient context is known, much less a complete system with central administration of the context management process.

A context management administrator is described in detail in U.S. Patent Application Serial No. 09/545,396, referred to above.

Context managers and context management administration software require communication from a user via a user interface. Conventional context managers and context management administrators therefore require a console or monitor and keyboard connected to the computer system on which they execute, in order for the user to communicate therewith. A context management administrator may communicate directly with one or more context managers residing on the same computer system. Alternatively, they may communicate through a network.

Likewise a context manager may execute on a computer system in common with the applications whose context is managed, or may execute on a remote computer system, communicating with the applications over a network.

A server appliance is a relatively new type of computing device. The server appliance is a network-connected server that provides a service to multiple client computers. A client requests the server to perform a specific task, such as returning a response to a database query. The server performs the task and returns the result of having performed the task back to the client.

However, unlike traditional computing servers that provide general-purpose platforms for a wide range of computing tasks, a server appliance is singular in purpose. A server appliance contains specialized software, and possibly specialized hardware, as well, to enable it to achieve its specialized purpose. Server appliances can therefore be optimized for the specific tasks that they may be designed to perform, thereby reducing the server cost and complexity as compared to the cost and complexity of general-purpose servers.

Conventional commercially available server appliances include print server appliances, whose only function is to queue print jobs and route them to appropriate printers; web server appliances, whose only function is to host a single web site or small group of web sites; electronic mail server appliances, whose only function is to host electronic mail services; and, file server appliances, whose only function is to centrally store and retrieve computer files.

The computing hardware on which server appliances are built conventionally includes a central processing unit, memory and long term data storage, all packaged within a single unit. The unit conventionally has only a power supply input and a network input/output (I/O) port. The network I/O port may connect the server appliance to a computer network using any conventional networking hardware, including but not limited to a modem connection, an Ethernet connection, a universal serial bus (USB) connection, etc.

The user controls on a server appliance are conventionally very simple. There may be no controls at all, only a power connection and a network connection, as noted above. Alternatively, there may simply be an on/off switch.

Clinical context working group: "The Clinical Context Object Workgroup: Its Standard and Methods" Internet Document: Clinical Context Object Working Group White Paper, [Online] 16 February 1998 (1998-02-16), XP002154044 describes a cooperative interaction among independent health care applications from the point of use, including the interaction of software and component interfaces for context management of a plurality of applications, including a context manager implementation and several application implementations. Common content data are accessed by the application implementations to uniformly effect the behaviour or operation of the multiple health care applications. It is considered necessary to manage the context so that the user has a way of controlling it, and so that applications have a way of coordinating their behaviour as the context changes. Context data are implemented by the common clinical context manager and are used by the applications to set or get the values for elements that comprise the clinical context. A mapper interface is implemented by an application that wants to provide the translation service for multiple ID's.

### SUMMARY OF THE INVENTION

The present invention as set out in claims 1 and 11 overcomes problems with the conventional approaches to hosting context management and context management administration software by providing, according to various aspects and embodiments thereof, a turnkey system providing context management through a network. Embodiments of the invention can bootstrap themselves into operation after only being connected to a power supply and network.

One embodiment of the invention in a context management server appliance includes a computer system and memory executing a stored set of instruction. The computer system has a power supply input and a network input/output (I/O) port. The memory includes a memory in which is stored a set of instructions defining a context management server which delivers context management information to client applications and a memory in which is stored a set of instructions defining a software interface for administering the context management server over the network using a general-purpose client interface. According to another embodiment of the invention, the context management server appliance further includes a memory in which is stored configuration information for the context management server, whereby the context management server can bootstrap without requiring user intervention. When such an embodiment can bootstrap independently of user intervention, there may also be a memory in which is stored a set of instructions which when executed connect the server appliance to the network absent user intervention. According to another embodiment of the context management server appliance there may be included a memory in which is stored a set of instructions which when executed balance a processing load on the server appliance with a processing load on another server appliance. According to yet another embodiment of the invention, the context management server appliance includes a memory in which is stored a set of instructions which when executed transfers a processing load from a failed server appliance to another server appliance.

Some embodiments of the invention may use servers, which communicate using a standard protocol such as the Hypertext Transport Protocol used in the World Wide Web of the global computer network, the Internet. In such an embodiment, applications can access the context management server through a World Wide Web universal resource locator (URL) on the global Internet. Such a URL need not provide access by a user of a conventional browser client to any information, but may be accessible only to application programs whose context are managed. The protection may use user ID and password, encryption and other trusted transaction systems known in the art.

### BRIEF DESCRIPTION OF THE DRAWINGS

In the drawings, in which like reference designations indicate like elements:
Fig. 1 is a block diagram of a system of context management applications and the context manager operating through a network;
Fig. 2 is a block diagram of one embodiment of a context management server appliance;
Fig. 3 is a block diagram of another embodiment of the invention constructed using two server appliances; and
Fig. 4 is a block diagram of yet another embodiment of the invention with built in load sharing and fault tolerant features.

### DETAILED DESCRIPTION

The invention will be better understood upon reading the following detailed description of some embodiments and aspects thereof in connection with the figures.

In accordance with some embodiments of the invention, a software agent acting on behalf of the user, referred to as a *Context Manager* (CM), enables applications to establish and maintain a common context on behalf of a user. The context may be organized in a variety of ways. For example, in the healthcare industry, the Health Level Seven Context Management Standard represents clinical context as a set of context subjects. Each subject represents a real-world entity, such as a patient or clinical provider, or a real-world concept, such as a clinical encounter, order, or disease state. Context management is explained in some detail in U.S. Patent Application Serial No. 09/545,396. noted above.

When a user performs a relevant application gesture, such as selecting a patient from a list of patients, the application informs the context manager of this fact. The context manager is then responsible for conveying to the other applications that a patient has been selected. Information that identifies the patient is conveyed via the context manager. All of the applications in use then tune their data displays to the selected patient.

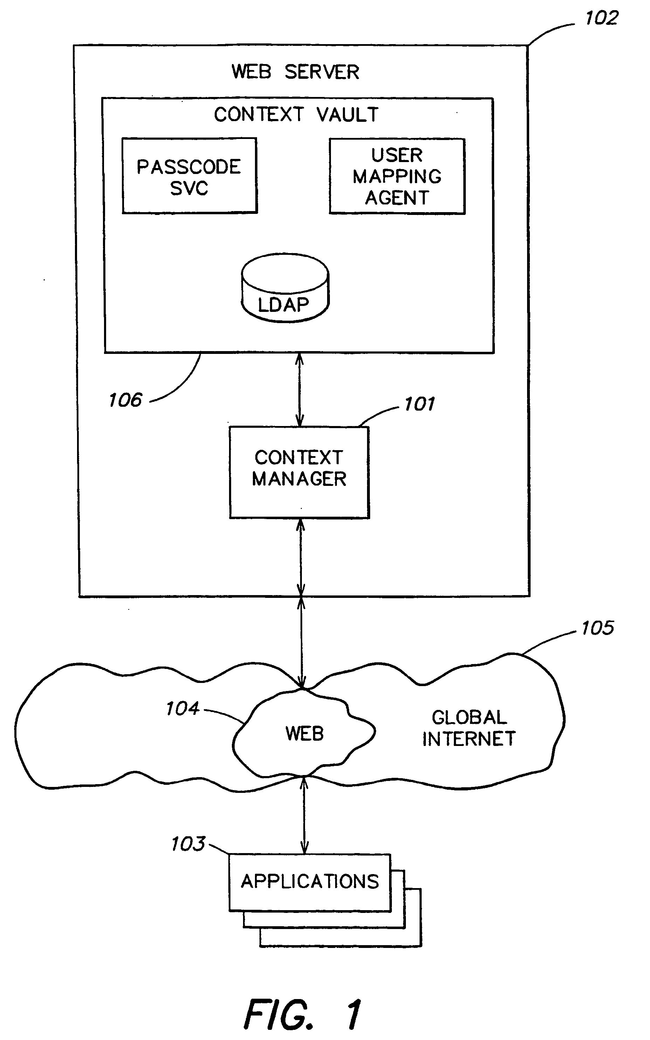

In accordance with one aspect of the invention, shown in Fig. 1, the CM resides and executes on a computer, for example web server 102, separate from the application 103 managed. The applications send information to and receive information from the CM through the World Wide Web 104 over the global Internet 105. Communication is effected using conventional protocols such as TCP/IP and HTTP, as needed.

The Web server 102 shown may be distributed over one or more computers providing the functions of the CM 101 and a context vault 106 providing passcode services and a user-mapping agent (UMA). These functions are all described in detail in U.S. Patent Application Serial No. 09/545,396, noted above.

A World Wide Web server provides the only application interface needed for the applications to access the CM and the supporting Context Vaults.

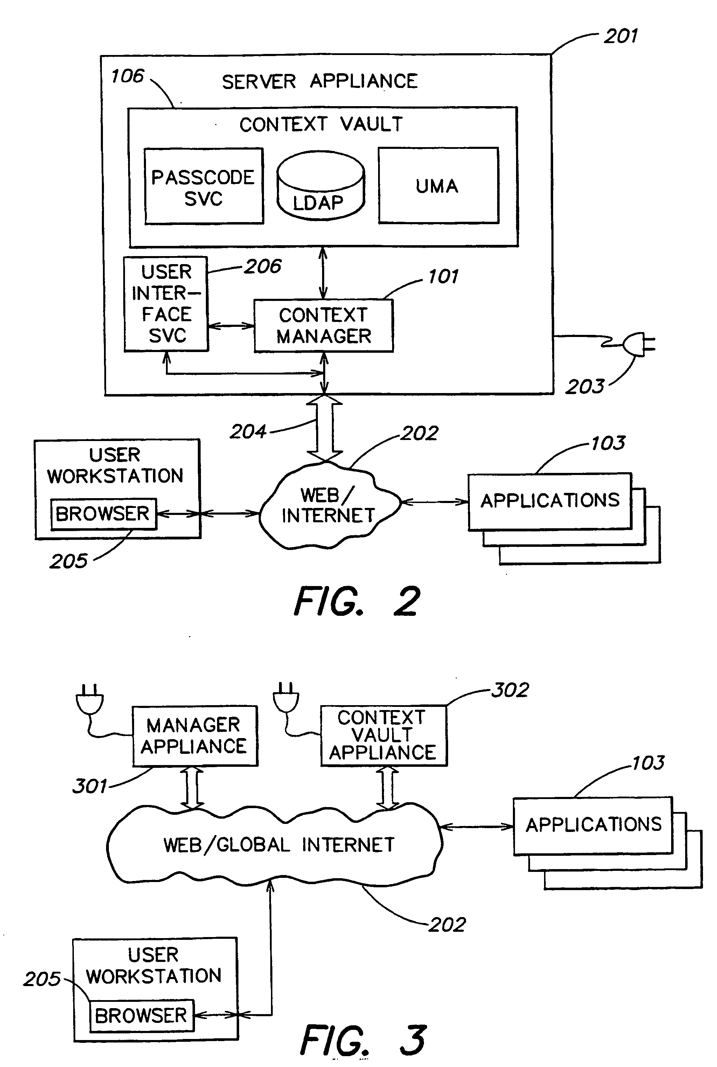

In accordance with another aspect of the invention, as shown in Fig. 2, the context manager 101 resides on the server appliance 201, but tracks and maintains a user's context as established by the user upon a particular computer running an application 103. A single context manager 101 may service one, or several, computers and applications. However, each computer perceives that it is interacting with a single context manager 101.

A network connection 202 between each user's computer and the server appliance enables the necessary communication. The network which the server appliance of Fig. 2 is connected to may be the global Internet, for example, or another wide area or local area network. In addition to the context manager 101, the server appliance 201 may contain additional service modules that support the context manager in performing its tasks. As shown in Fig. 2, the appliance may provide the CM 101 and Context Vault 106 functions.

The physical controls for a server appliance are generally limited in nature, and in the simplest case may consist only of an on-off switch. A power connection 203 and a network connection 204 complete this simplest of physical configurations. Devoid of a keyboard, mouse, and other traditional user-interface devices, any interaction with the appliance for the purposes of installation, configuration or maintenance is effected via remote applications that communicate with the appliance through its network connection, for example using conventional browser 205 client software. In such a configuration, the browser 205 would be directed to a particular location 206 in the Web site supported by the appliance, at which a configuration program can be accessed and controlled through an interface displayed by the browser 205 in response to messages from the configuration program 206.

The context management server appliance can be implemented in a variety of ways. One way is to adapt an existing server appliance device by creating and installing the necessary context management software and complementary support tools. The network connection to the Internet would be based upon the TCP/IP protocol upon which a higher-level communication protocol such as HTTP might be used, as mentioned above.

The specific network messages that pertain to context management can be represented in a variety of ways. One example is to define messages using the syntax of HTTP, such that each message represents a specific action for the server to process. For example, an application might send a specific message when it wants to change the data for the current context. It would send a different message if it wanted to obtain the data for the current context.

The functionality of context management can be partitioned, according to another aspect of the invention shown in Fig. 3. In this exemplary partitioning, the CM and Context Vault are each configured to execute on their own server appliance 301 and 302, respectively. The server appliances 301, 302 then communicate with each other through the network 202 to which they are connected. Applications 103 whose context is managed communicate with the CM using TCP/IP and HTTP, for example, as before.

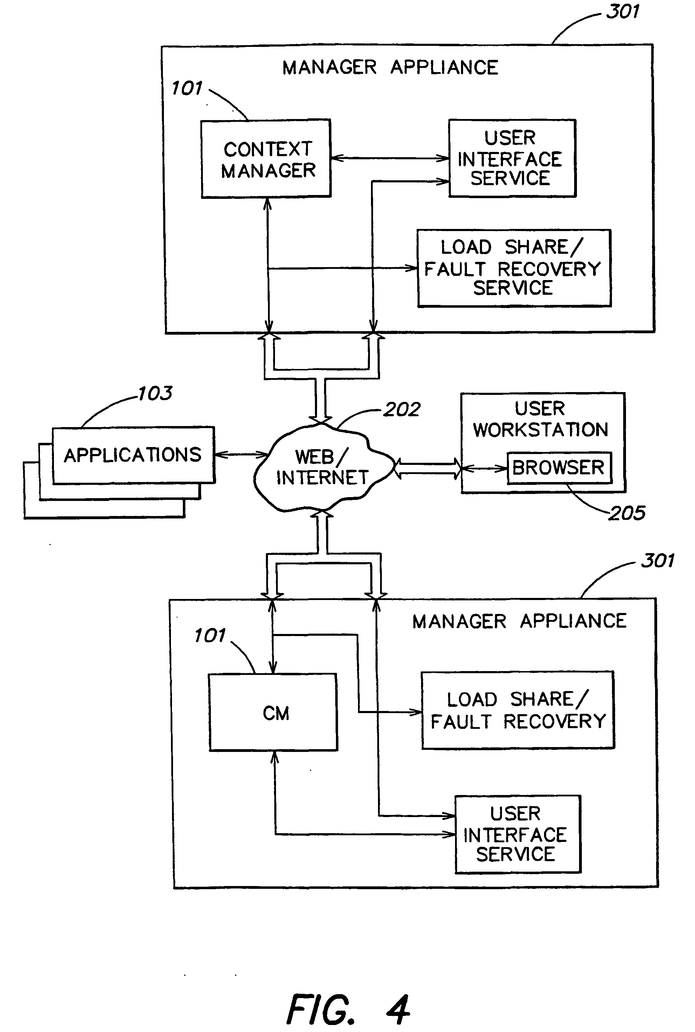

As shown in Fig. 4, plural manager appliances can be connected to the network. When an application addresses a context manager, the communication may be mediated by a service which ensures that the work load of the plurality of context managers is fairly distributed. The load sharing service itself may be distributed amongst the various context managers which communicate with each other and with the managed applications through the network. When a context manager fails or is taken off line for service, the same or a similar service can ensure that context management tasks are suitably redistributed amongst the remaining context management server appliances.

Server appliances embodying aspects of the invention do not merely serve back to the managed applications stored data or simply process input messages. These server appliances perform all of the complex CM functions described in U.S. Patent Application Serial No. 09/545,396, as well as serving the result of that activity to the managed applications.

This invention has been motivated by the application of context management to healthcare. However, context management, and therefore a context management server appliance, can be applied to many industries, and as such this invention is not limited to healthcare.

A network connection between each user's computer and the server appliance, and/or between other general-purpose servers and the server appliance, enables the necessary communication. In addition, the server appliance may contain additional modules that enable the server to be remotely configured and supported.

In the healthcare field, the use of a server appliance for hosting the services relating to maintaining a Master Patient Index, and for hosting services relating to Coding data, is particularly unique.

In general, a Master Patient Index (MPI) implements an application service wherein the myriad of identifiers typically assigned by a healthcare organization to represent each person known to the organization are reconciled. This reconciliation enables the unambiguous correlation of information about a person who is represented by different identifiers in different electronic and paper systems. An MPI typically maintains additional descriptive information about each person. This information, usually referred to as demographics data, includes the person's full name, address, telephone number, etc.

There are currently many MPI software products, each implementing various algorithms for correlating person identifiers. However, these products are all deployed on general-purpose servers. In embodiments of this invention, the MPI is deployed within a server appliance, thereby providing an optimized, cost-effective, easier-to-maintain, information utility for the healthcare enterprise.

Another type of healthcare server appliance is a Coding server appliance. In general, coding involves the representation of data or concepts as a numeric value, even if the data or concept is not numeric in nature. The numeric representation enables the data or concept to be annotated in terms of its semantic meaning. This enables the electronic interpretation of meaning and facilitates electronically computed deductions, rules, constraints, and comparisons.

For example, the healthcare industry has defined a variety of schemes for representing medications using coded values. This enables physicians to precisely indicate (either manually or electronically) the particular medication issued, independent of the medication's trade name. Once the coded value is entered, systems can check whether the medication has been appropriately administered, determine how much to charge to the patient's bill, etc.

There are currently many Coding software products, each implementing various algorithms for annotating data with coded values. However these products are all deployed on general-purpose servers. In this invention, the Coding is deployed within a server appliance, thereby providing an optimized, cost-effective, easier-to-maintain, information utility for the healthcare enterprise.

Embodiments of a healthcare MPI or Coding server appliance can be implemented in a variety of ways. One way is to adapt an existing server appliance device by creating and installing the necessary software and complementary support tools. The network connection is most likely based upon the TCP/IP protocol upon which a higher-level communication protocol such as HTTP might be used.

The specific network messages that pertain to context management can be represented in a variety of ways. One example is to define messages using the syntax of HTTP, such that each message represents a specific action for the server to process. For example, an application might send a specific message to an MPI server appliance when it wants to determine if two identifiers represent the same person. It would send a different message if it wanted to obtain demographics data for a person.

The invention has been illustrated by the foregoing description of a number of aspects and embodiments thereof. Numerous variations contemplated as within the scope of the invention as defined by the appended claims should now be apparent to those skilled in the art. The invention should therefore not be limited by the foregoing description, but rather by the properly construed claims, which follow.

## Claims

1. A context management server, comprising:
a computer system (201) having a power supply input and a network input/output (I/O) port; and
memory means in which is stored a set of instructions defining the context management server (101), adapted to deliver context management information to a plurality of client applications (103) executing on a common workstation through network connections (204) of a network (202).

2. The context management server of claim 1, wherein the context management server is constituted by the server appliance.

3. The context management server of claim 1, wherein the context management server is constituted by the Web server.

4. The context management server of claim 1, further comprising:
memory means in which is stored configuration information for the context management server, whereby the context management server can bootstrap without requiring user intervention.

5. The context management server of claim 2, further comprising:
memory means in which is stored a set of instructions which when executed connect the server to the network absent user intervention.

6. The context management server of claim 1, further comprising:
memory means in which is stored a set of instructions which when executed balance a processing load on the server with a processing load on another server.

7. The context management server of claim 1, further comprising:
memory means in which is stored a set of instructions which when executed transfers a processing load from a failed server to another server.

8. The context management server of claim 1, further comprising:
memory means in which is stored a Master Patient Index.

9. The context management server of claim 1, further comprising:
memory means in which is stored a healthcare coding index.

10. The context management server of claim 1, further comprising:
memory means in which is stored a set of instructions defining a context vault accessible to the context management server, wherein the context vault is accessible to the context management server through a network.

11. A method for context management over a network, comprising:
delivering context management information to a plurality of client applications (103) executing on a common workstation through network connections (204) of a network (202) based on a set of instructions stored in memory means and defining a context management server (101).

12. The method of claim 11, wherein the context management server is constituted by the server appliance.

13. The method of claim 11, wherein the context management server is constituted by the Web server.

14. The method of claim 11, including receiving, on the server, via the network, a first network message, in accordance with a network communication protocol,
containing information pertaining to a context management action;
performing, on the server, an act pertaining to the context management action; and
sending, from the server, via the network, a second network message, in accordance with the network communication protocol, containing information pertaining to the context management action. ,

15. The method of claim 11, wherein performing the act pertaining to context management comprises performing a processing function in a context manager implemented on the server.

16. The method of claim 11, wherein performing the act pertaining to context management comprises performing a processing function in a context vault implemented on the server.

17. The method of claim 11, wherein performing the act pertaining to context management comprises performing processing functions, in each of a context manager and a context vault, each of which is implemented on the server.

18. The method of claim 11, further comprising determining whether to use the server or another, similarly-configured network appliance, based on load sharing considerations.

19. The method of claim 11, wherein receiving and sending the network messages is done using the TCP/IP protocol.

20. The method of claim 11, wherein receiving and sending the network messages is done using the HTTP protocol.

21. The method of claim 11, wherein the context management server is constituted by the server appliance, further comprising adapting an existing server appliance for use as a context management server appliance.

22. The method of claim 21, wherein adapting the existing server appliance comprises installing context management software onto the existing server appliance.

23. The method of claim 11, further comprising sending information, from a context client, to the server, over the network.

24. The method of claim 11, further comprising receiving information, from the server, on a context client, over the network.

25. The method of claim 11, wherein the context management action pertains to patient medical care.

26. The method of claim 25, wherein the context management action pertaining to patient medical care comprises an action on a master patient index (MPI).

27. The method of claim 25, wherein the context management action pertaining to patient medical care is in accordance with a healthcare industry standard.

28. The method of claim 11, further comprising coupling the server appliance to a Web server, said Web server managing communication between the server appliance and other elements coupled to the network.

29. The method of claim 28, further comprising running software on the Web server capable of supporting Web browser applications and an interface to client applications.

30. The method of claim 11, wherein the network is any of: a wide area network, a local area network and the Internet.

31. The method of claim 11, further comprising performing a coding act wherein context data is represented by corresponding numeric data.

32. A context management system, comprising:
a context management server according to claim 1; and
a network, coupled to the context management server via the network connection, said network carrying information pertaining to context management actions to and from the context management server.

33. The context management system of claim 32, wherein the context management server is constituted by the server appliance.

34. The context management system of claim 32, wherein the context management server is constituted by the Web server.

35. The context management system of claim 32, wherein the context management server is a context management server appliance implemented on an existing server appliance.

36. The context management system of claim 32, further comprising a load manager for distributing context management loads between a plurality of context management servers.

37. The context management system of claim 32, wherein the network is any of: a wide area network, a local area network and the Internet.

38. The context management system of claim 32, wherein the Web server is for managing communication between the context management server and other elements coupled to the network.

39. The context management system of claim 38, further comprising software for supporting a Web browser and a client application interface.

40. The context management system of claim 32, further comprising a master patient index (MPI).

41. The context management system of claim 32, wherein the context management server comprises a context manager.

42. The context management system of claims 32, wherein the context management server comprises a context vault.

43. The method context management system of claim 32, wherein the context data are represented with coded values.

## Patentansprüche

1. Ein Kontextmanagementserver, umfassend:
ein Computersystem (201), dass einen Netzanschluss und einen Netzwerks-Eingabe/Ausgabe Anschluss aufweist; und
eine Speichereinrichtung, in der ein Satz von den Kontextmanagementserver (101) definierenden Instruktionen gespeichert ist, wobei dieser zum Liefern von Kontextmanagementinformationen an eine Vielzahl von Client-Anwendungen (103) angepasst ist, die auf einer gemeinsamen Arbeitsstation über Netzwerkverbindungen (204) eines Netzwerks (202) ausgeführt werden.

2. Der Kontextmanagementserver nach Anspruch 1, wobei der Kontextmanagementserver durch das Servergerät gebildet ist.

3. Der Kontextmanagementserver nach Anspruch 1, wobei der Kontextmanagementserver durch den Netzserver gebildet ist.

4. Der Kontextmanagementserver nach Anspruch 1, ferner umfassend:
Speichereinrichtung, in der Konfigurationsinformationen für den Kontextmanagementserver gespeichert sind, wobei der Kontextmanagementserver urladen bzw. bootstrap kann, ohne Bedienereingriff zu benötigen.

5. Der Kontextmanagementserver nach Anspruch 2, ferner umfassend:
Speichereinrichtung, in der ein Satz von Instruktionen gespeichert ist, die bei Ausführung den Server mit dem Netzwerk ohne Eingriff des Benutzers verbindet.

6. Der Kontextmanagementserver nach Anspruch 1, ferner umfassend:
Speichereinrichtung, in der ein Satz von Instruktionen gespeichert ist, die bei Ausführung die Bearbeitungsauslastung auf dem Server mit der Bearbeitungsauslastung auf einem anderen Server ausgleicht.

7. Der Kontextmanagementserver nach Anspruch 1, ferner umfassend:
Speichereinrichtung, in der ein Satz von Instruktionen gespeichert ist, die bei Ausführung eine Bearbeitungsauslastung von einem fehlerhaften Server auf einen anderen Server transferiert.

8. Der Kontextmanagementserver nach Anspruch 1, ferner umfassend:
Speichereinrichtung, in der ein Hauptpatientenindex gespeichert ist.

9. Der Kontextmanagementserver nach Anspruch 1, ferner umfassend:
Speichereinrichtung, in der ein Gesundheitspflegekodierungsindex gespeichert ist.

10. Der Kontextmanagementserver nach Anspruch 1, ferner umfassend:
Speichereinrichtung, in der ein Satz von Instruktionen gespeichert ist, die ein für den Kontextmanagementserver erreichbare Kontextsicherheitseinrichtung definiert, wobei die Kontextsicherheitseinrichtung für den Kontextmanagementserver durch ein Netzwerk erreichbar ist.

11. Ein Verfahren für Kontextmanagement über ein Netzwerk, umfassend:
Liefern von Kontextmanagementinformationen an eine Vielzahl von Client-Anwendungen (103), die auf einer gemeinsamen Arbeitsstation über Netzwerkverbindungen (204) auf ein Netzwerk (202) ausgeführt werden, die auf einen Satz von in der Speichereinrichtung gespeicherten Instruktionen basiert und einen Kontextmanagementserver (101) definiert.

12. Das Verfahren nach Anspruch 11, wobei der Kontextmanagementserver durch das Servergerät gebildet ist.

13. Das Verfahren nach Anspruch 11, wobei der Kontextmanagementserver durch den Netzserver gebildet ist.

14. Das Verfahren nach Anspruch 11, umfassend das Empfangen einer ersten Netzwerknachricht über das Netzwerk auf dem Server in Übereinstimmung mit einem Netzwerkkommunikationsprotokoll, das Informationen betreffend einer
Kontextmanagementaktion beinhaltet;
Ausführen einer Aktion auf dem Server betreffend der Kontextmanagementaktion; und
Senden einer zweiten Netzwerknachricht vom Server über das Netzwerk in Übereinstimmung mit dem Netzwerkkommunikationsprotokoll, das Informationen betreffend einer Kontextmanagementaktion beinhaltet.

15. Das Verfahren nach Anspruch 11, wobei das Ausführen einer Aktion betreffend des Kontextmanagement das Ausführen einer Bearbeitungsfunktion in einem in einem Server eingebauten Kontext Manager umfasst.

16. Das Verfahren nach Anspruch 11, wobei Ausführen einer Aktion betreffend des Kontextmanagement das Ausführen einer Bearbeitungsfunktion in einer in einem Server eingebauten Kontextsicherheitseinrichtung umfasst.

17. Das Verfahren nach Anspruch 11, wobei Ausführen einer Aktion betreffend des Kontextmanagement das Ausführen von Bearbeitungsfunktionen in einem Kontextmanager und einer Kontextsicherheitseinrichtung umfasst, wobei jedes in einem Server eingebaut ist.

18. Das Verfahren nach Anspruch 11, ferner umfassend, basiert auf Auslastungsteilungsentscheidungen, Feststellen, ob der Server oder ein anderes, vergleichbar konfiguriertes Netzwerkgerät verwendet werden soll.

19. Das Verfahren nach Anspruch 11, wobei Empfangen und Senden der Netzwerknachrichten durch Verwendung des TCP/IP Protokolls erfolgt.

20. Das Verfahren nach Anspruch 11, wobei Empfangen und Senden der Netzwerknachrichten durch Verwendung des HTTP Protokolls erfolgt.

21. Das Verfahren nach Anspruch 11, wobei der Kontextmanagementserver durch das Servergerät gebildet ist, ferner umfassend das Anpassen eines bestehenden Servergeräts zum Verwenden als ein Kontextmanagementservergerät.

22. Das Verfahren nach Anspruch 21, wobei das Anpassen des bestehenden Servergeräts das Installieren von Kontextmanagementsoftware auf das bestehende Servergerät umfasst.

23. Das Verfahren nach Anspruch 11, ferner umfassend das Senden von Informationen von einem Kontext-Client an einen Server über das Netzwerk.

24. Das Verfahren nach Anspruch 11, ferner umfassend das Empfangen von Informationen von einem Server an einen Kontext-Client über das Netzwerk.

25. Dass Verfahren nach Anspruch 11, wobei die Kontextmanagementaktion medizinische Pflege von Patienten betrifft.

26. Dass Verfahren nach Anspruch 25, wobei die medizinische Pflege von Patienten betreffende Kontextmanagementaktion eine Aktion am Hauptpatientenindex (MPI) umfasst.

27. Das Verfahren nach Anspruch 25, wobei die medizinische Pflege von Patienten betreffende Kontextmanagementaktion in Übereinstimmung ist mit einem Industriestandard der Gesundheitspflege.

28. Das Verfahren nach Anspruch 11, ferner umfassend das Koppeln des Servergeräts an einen Netzserver, wobei der Netzserver die Kommunikation zwischen dem Servergerät und andere an das Netzwerk gekoppelte Elemente regelt.

29. Das Verfahren nach Anspruch 28, ferner umfassend das Laufen von Software auf dem Netzserver, die in der Lage ist, Netzbrowseranwendungen und eine Schnittstelle für Client-Anwendungen zu unterstützen.

30. Das Verfahren nach Anspruch 11, wobei das Netzwerk jedes ist von: einem Fernnetzwerk, einem lokalen Netzwerk und dem Internet.

31. Das Verfahren nach Anspruch 11, ferner umfassend das Durchführen eines Kodierungsvorganges, wobei Kontextdaten durch korrespondierende numerische Daten repräsentiert werden.

32. Ein Kontextmanagementsystem, umfassend:
einen Kontextmanagementserver nach Anspruch 1; und
ein Netzwerk, das über die Netzwerkverbindung an den Kontextmanagementserver gekoppelt ist, wobei das Netzwerk Informationen betreffend der Kontextmanagementaktionen zu und vom Kontextmanagementserver beinhaltet.

33. Das Kontextmanagementsystem nach Anspruch 32, wobei der Kontextmanagementserver durch das Servergerät gebildet ist.

34. Das Kontextmanagementsystem nach Anspruch 32, wobei der Kontextmanagementserver durch den Netzserver gebildet ist.

35. Das Kontextmanagementsystem nach Anspruch 32, wobei der Kontextmanagementserver ein auf einem vorhandenen Servergerät eingebautes Kontextmanagementservergerät ist.

36. Das Kontextmanagementsystem nach Anspruch 32, ferner umfassend einen Auslastungsmanager zum Verteilen von Kontextmanagementauslastungen zwischen einer Vielzahl von Kontextmanagementservern.

37. Das Kontextmanagementsystem nach Anspruch 32, wobei das Netzwerk jedes ist von: einem Fernnetzwerk, einem lokalen Netzwerk und dem Internet.

38. Das Kontextmanagementsystem nach Anspruch 32, wobei der Netzserver zum Regeln von Kommunikation zwischen dem Kontextmanagementserver und anderen an das Netzwerk gekoppelten Elementen ist.

39. Das Kontextmanagementsystem nach Anspruch 38, ferner umfassend Software zum Unterstützen eines Netzbrowsers und einer Kundenanwendungsschnittstelle.

40. Das Kontextmanagementsystem nach Anspruch 32, ferner umfassend einen Hauptpatientenindex (MPI).

41. Das Kontextmanagementsystem nach Anspruch 32, wobei der Kontextmanagementserver einen Kontextmanager umfasst.

42. Das Kontextmanagementsystem nach Anspruch 32, wobei der Kontextmanagementserver eine Kontextsicherheitseinrichtung umfasst.

43. Das Kontextmanagementsystem Verfahren nach Anspruch 32, wobei die Kontextdaten mit kodierten Werten repräsentiert sind.

## Revendications

1. Serveur de gestion de contexte, comprenant :
un système d'ordinateur (201) comportant une entrée d'alimentation et un port d'entrée/sortie de réseau (E/S), et
un moyen de mémoire dans lequel est mémorisé un jeu d'instructions définissant le serveur de gestion de contexte (101), conçu pour délivrer des informations de gestion de contexte à une pluralité d'applications client (103) s'exécutant sur une station de travail commune par l'intermédiaire des connexions de réseau (204) d'un réseau (202).

2. Serveur de gestion de contexte selon la revendication 1, dans lequel le serveur de gestion de contexte est constitué d'un dispositif serveur.

3. Serveur de gestion de contexte selon la revendication 1, dans lequel le serveur de gestion de contexte est constitué par un serveur de réseau Web.

4. Serveur de gestion de contexte selon la revendication 1, comprenant en outre :
un moyen de mémoire dans lequel sont mémorisées des informations de configuration pour le serveur de gestion de contexte, grâce auxquelles le serveur de gestion de contexte peut exécuter un amorçage sans requérir une intervention de l'utilisateur.

5. Serveur de gestion de contexte selon la revendication 2, comprenant en outre :
un moyen de mémoire dans lequel est mémorisé un jeu d'instructions qui, lorsqu'elles sont exécutées, connectent le serveur au réseau en l'absence d'une intervention de l'utilisateur.

6. Serveur de gestion de contexte selon la revendication 1, comprenant en outre :
un moyen de mémoire dans lequel est mémorisé un jeu d'instructions qui, lorsqu'elles sont exécutées, équilibrent une charge de traitement sur le serveur avec une charge de traitement sur un autre serveur.

7. Serveur de gestion de contexte selon la revendication 1, comprenant en outre :
un moyen de mémoire dans lequel est mémorisé un jeu d'instructions qui, lorsqu'elles sont exécutées, transfèrent une charge de traitement d'un serveur en panne à un autre serveur.

8. Serveur de gestion de contexte selon la revendication 1, comprenant en outre :
un moyen de mémoire dans lequel est mémorisé un index maître des patients.

9. Serveur de gestion de contexte selon la revendication 1, comprenant en outre :
un moyen de mémoire dans lequel est mémorisé un index de codage de services médicaux.

10. Serveur de gestion de contexte selon la revendication 1, comprenant en outre :
un moyen de mémoire dans lequel est mémorisé un jeu d'instructions définissant un module sécurisé de contexte accessible au serveur de gestion de contexte, où le module sécurisé de contexte est accessible au serveur de gestion de contexte par l'intermédiaire d'un réseau.

11. Procédé destiné à une gestion de contexte sur un réseau, comprenant :
la délivrance d'informations de gestion dé contexte à une pluralité d'applications client (103) s'exécutant sur une station de travail commune par l'intermédiaire de connexions de réseau (204) d'un réseau (202) sur la base d'un jeu d'instructions mémorisé dans un moyen de mémoire et définissant un serveur de gestion de contexte (101).

12. Procédé selon la revendication 11, dans lequel le serveur de gestion de contexte est constitué par un dispositif serveur.

13. Procédé selon la revendication 11, dans lequel le serveur de gestion de contexte est constitué par un serveur de réseau Web.

14. Procédé selon la revendication 11, comprenant la réception, sur le serveur, par l'intermédiaire du réseau, d'un premier message de réseau, conforme à un protocole de communication de réseau, contenant des informations se rapportant à une activité de gestion de contexte,
l'exécution, sur le serveur, d'une action se rapportant à l'activité de gestion de contexte, et
l'envoi, depuis le serveur, par l'intermédiaire du réseau, d'un second message de réseau, conforme au protocole de communication de réseau, contenant des informations se rapportant à l'activité de gestion de contexte.

15. Procédé selon la revendication 11, dans lequel l'exécution de l'action se rapportant à la gestion de contexte comprend l'exécution d'une fonction de traitement dans un gestionnaire de contexte mis en oeuvre sur le serveur.

16. Procédé selon la revendication 11, dans lequel l'exécution de l'action se rapportant à une gestion de contexte comprend l'exécution d'une fonction de traitement dans un module sécurisé de contexte mis en oeuvre sur le serveur.

17. Procédé selon la revendication 11, dans lequel l'exécution de l'action se rapportant à une gestion de contexte comprend l'exécution de fonctions de traitement, dans chacun d'un gestionnaire de contexte et d'un module sécurisé de contexte, dont chacun est mis en oeuvre sur le serveur.

18. Procédé selon la revendication 11, comprenant en outre la détermination du fait qu'il faut utiliser le serveur ou un autre dispositif de réseau configuré de manière similaire, sur la base de considérations de partage de charges.

19. Procédé selon la revendication 11, dans lequel la réception et l'envoi des messages de réseau se font en utilisant le protocole TCP/IP.

20. Procédé selon la revendication 11, dans lequel la réception et l'envoi des messages de réseaux se font en utilisant le protocole HTTP.

21. Procédé selon la revendication 11, dans lequel le serveur de gestion de contexte est constitué par le dispositif serveur, comprenant en outre l'adaptation d'un dispositif serveur existant pour l'utiliser comme dispositif serveur de gestion de contexte.

22. Procédé selon la revendication 21, dans lequel l'adaptation du dispositif serveur existant comprend l'installation d'un logiciel de gestion de contexte sur le dispositif serveur existant.

23. Procédé selon la revendication 11, comprenant en outre l'envoi d'informations, depuis un client de contexte, au serveur, sur le réseau.

24. Procédé selon la revendication 11, comprenant en outre la réception d'informations, depuis le serveur, sur un client de contexte, sur le réseau.

25. Procédé selon la revendication 11, dans lequel l'activité de gestion de contexte se rapporte à des soins médicaux de patients.

26. Procédé selon la revendication 25, dans lequel l'activité de gestion de contexte se rapportant à des soins médicaux de patients comprend une activité sur un index maître de patients (MPI).

27. Procédé selon la revendication 25, dans lequel l'activité de gestion de contexte se rapportant à des soins médicaux de patients se fait conformément à une norme de l'industrie pour les services médicaux.

28. Procédé selon la revendication 11, comprenant en outre le couplage du dispositif serveur à un serveur de réseau Web, ledit serveur de réseau Web gérant une communication entre le dispositif serveur et d'autres éléments reliés au réseau.

29. Procédé selon la revendication 28, comprenant en outre l'exécution d'un logiciel sur le serveur de réseau Web permettant d'accepter des applications de navigateurs de réseau Web et une interface vers des applications client.

30. Procédé selon la revendication 11, dans lequel le réseau est l'un quelconque de : un réseau à grande distance, un réseau local et le système Internet.

31. Procédé selon la revendication 11, comprenant en outre l'exécution d'une action de codage dans laquelle des données de contexte sont représentées par des données numériques correspondantes.

32. Système de gestion de contexte, comprenant :
un serveur de gestion de contexte selon la revendication 1, et
un réseau, relié au serveur de gestion de contexte par l'intermédiaire de la connexion de réseau, ledit réseau transportant des informations se rapportant à des activités de gestion de contexte vers et depuis le serveur de gestion de contexte.

33. Système de gestion de contexte selon la revendication 32, dans lequel le serveur de gestion de contexte est constitué par le dispositif serveur.

34. Système de gestion de contexte selon la revendication 32, dans lequel le serveur de gestion de contexte est constitué par le serveur de réseau Web.

35. Système de gestion de contexte selon la revendication 32, dans lequel le serveur de gestion de contexte est un dispositif serveur de gestion de contexte mis en oeuvre sur un dispositif serveur existant.

36. Système de gestion de contexte selon la revendication 32, comprenant en outre un gestionnaire de charge destiné à répartir des charges de gestion de contexte entre une pluralité de serveurs de gestion de contexte.

37. Système de gestion de contexte selon la revendication 32, dans lequel le réseau est l'un quelconque de :
un réseau à grande distance, un réseau local et le système Internet.

38. Système de gestion de contexte selon la revendication 32, dans lequel le serveur de réseau Web est destiné à gérer une communication entre le serveur de gestion de contexte et d'autres éléments reliés au réseau.

39. Système de gestion de contexte selon la revendication 38, comprenant en outre un logiciel destiné à soutenir un navigateur de réseau Web et une interface d'application client.

40. Système de gestion de contexte selon la revendication 32, comprenant en outre un index maître de patients (MPI).

41. Système de gestion de contexte selon la revendication 32, dans lequel le serveur de gestion de contexte comprend un gestionnaire de contexte.

42. Système de gestion de contexte selon la revendication 32, dans lequel le serveur de gestion de contexte comprend un module sécurisé de contexte.

43. Système de gestion de contexte selon la revendication 32, dans lequel les données de contexte sont représentées par des valeurs codées.
